# EUROPEAN PATENT SPECIFICATION

(11) **EP 4 026 561 B1**
(45) Date of publication and mention of the grant of the patent: **05.08.2026**
(21) Application number: 20837486.8
(22) Date of filing: 12.06.2020
(51) Int. Cl.: A61K 31/573, A61K 45/06, A61P 17/06

(54) **NEW APPLICATION OF CHEMOKINE RECEPTOR CCR6 INHIBITOR IN PREVENTING RECURRENCE OF PSORIASIS**
NEUE ANWENDUNG DES CHEMOKINREZEPTOR-CCR6-INHIBITORS ZUR VORBEUGUNG VON REZIDIVIERENDER PSORIASIS
NOUVELLE APPLICATION DE L'INHIBITEUR DU RÉCEPTEUR DE CHIMIOKINE CCR6 DANS LA PRÉVENTION DE LA RÉCURRENCE DU PSORIASIS

(30) Priority: 08.07.2019 CN 201910612157
(43) Date of publication of application: 13.07.2022
(73) Proprietor: Ruijin Hospital, Shanghai Jiaotong University School of Medicine, Shanghai 200025 (CN)
(72) Inventor: ZHENG, Jie, Shanghai 200025 (CN); LIU, Na, Shanghai 200025 (CN); QIN, Hui, Shanghai 200025 (CN); LI, Xia, Shanghai 200025 (CN); XUE, Feng, Shanghai 200025 (CN); CHEN, Lihong, Shanghai 200025 (CN); WANG, Lanqi, Shanghai 200025 (CN); QUAN, Sheng, Shanghai 200025 (CN); ZHANG, Li, Shanghai 200025 (CN)
(74) Representative: Santoro, Sofia
(86) International application number: PCT/CN2020/095956
(87) International publication number: WO 2021/004229

(56) References cited:
- WO-A1-2012/000906
- WO-A1-2013/184218
- WO-A1-2015/084842
- WO-A1-2016/059253
- WO-A1-2017/087607
- WO-A2-02/26764
- WO-A2-2005/095953
- WO-A2-2019/136370
- CN-A- 103 588 697
- MABUCHI TOMOTAKA ET AL: "CCR6 Is Required for Epidermal Trafficking of [gamma][delta]-T Cells in an IL-23-Induced Model of Psoriasiform Dermatitis", JOURNAL OF INVESTIGATIVE DERMATOLOGY, vol. 133, no. 1, 1 January 2013 (2013-01-01), NL, pages 164 - 171, XP093089281, ISSN: 0022-202X, DOI: 10.1038/jid.2012.260
- HEDRICK M N ET AL: "CCR6 as a possible therapeutic target in psoriasis", EXPERT OPINION ON THERAPEUTIC TARGETS, INFORMA HEALTHCARE, UK, vol. 14, no. 9, 1 September 2010 (2010-09-01), pages 911 - 922, XP008176873, ISSN: 1472-8222, DOI: 10.1517/14728222.2010.504716
- LEE CHIH-HUNG ET AL: "Pathophysiology of chemokines and chemokine receptors in dermatological science: A focus on psoriasis and cutaneous T-cell lymphoma", DERMATOLOGICA SINICA, vol. 30, no. 4, 2012, pages 128 - 135, XP028960664, ISSN: 1027-8117, DOI: 10.1016/J.DSI.2012.08.004
- IRÈNE GALLAIS SÉRÉZAL, HOFFER ELENA, IGNATOV BORISLAV, MARTINI ELISA, ZITTI BEATRICE, EHRSTRÖM MARCUS, EIDSMO LIV: "A skewed pool of resident T cells triggers psoriasis-associated tissue responses in never-lesional skin from patients with psoriasis", JOURNAL OF ALLERGY AND CLINICAL IMMUNOLOGY, vol. 143, no. 4, 13 August 2018 (2018-08-13), pages 1444 - 1454, XP055771746, ISSN: 0091-6749, DOI: 10.1016/j.jaci.2018.08.048

## Description

### Technical field

The present invention relates to the field of biomedicine, and in particular, to a new application of a chemokine receptor CCR6 inhibitor in preventing recurrence of psoriasis.

### Background

Psoriasis is a typical common inflammatory skin disease, with an incidence of about 2%-3% worldwide, and recurrence is its characteristic. Plaque type is the most common type of psoriasis, accounting for about 90% of all psoriasis patients, and appears as well-defined papulosquamous erythema, often symmetrically distributed on the scalp, extremities, and trunk.

The pathogenesis of psoriasis is currently unclear. Genetic susceptibility, environmental triggers, damage to skin barrier function, and immune system dysfunction are all involved. Different cells are involved in different stages of disease occurrence, development, stability, and recurrence. Classical treatments for psoriasis include topical drugs for external use such as emollients, glucocorticoids, and vitamin D derivatives for mild to moderate psoriasis, and systemic treatments such as phototherapy, methotrexate, cyclosporine, and acitretin for moderate to severe psoriasis. The main immunological events in the occurrence of psoriasis are the activation of skin keratinocytes by inflammatory factors such as TNF-α, IL-23 and IL17. It has been reported that the key pathogenic product for psoriasis is IL-17. Thus, the method of treatment of psoriasis that targets cytokines has emerged.

Although biological agents are a major innovation in treatment of psoriasis, a considerable number of patients, who has achieved disease remission after initial administration of the biological agents, encounter a gradually declined efficacy as the treatment time extends, thereby leading to recurrence of disease, and some even progress to more severe psoriasis types such as pustular, erythrodermic, and arthritic psoriasis. Among the above-mentioned treatment drugs, glucocorticoids for topical use have the longest history and are the most widely used. The recurrence after stopping administration as well as the recurrence and aggravation during the continuous administration are much distressing to both physicians and patients. Therefore, for the treatment of psoriasis, whether it is a traditional external preparation such as a glucocorticoid or an emerging biological preparation, the efficacy will decline as treatment time extends. After a short period of remission, most patients relapse ranging from a few weeks to a few months and some even have worsen symptoms.

However, the mechanisms underlying psoriasis recurrence remain unclear.

Therefore, there is an urgent need in the art to develop a therapy to prevent the recurrence of psoriasis in the treatment of psoriasis.

### Summary of the invention

The purpose of the present invention is to provide a therapy for preventing the recurrence of psoriasis in the treatment of psoriasis.

In the first aspect of the present invention, it provides the use of a chemokine receptor CCR6 inhibitor for preparing a formulation or composition, which is administered to a subject to prevent recurrence of psoriasis.

In another preferred embodiment, the subject is a mammal.

In another preferred embodiment, the mammal comprises a human or a non-human mammal.

In another preferred embodiment, the non-human mammal comprises: a rodent (e.g., rats, mice), a primate (e.g., monkeys).

In another preferred embodiment, the subject is a patient with psoriasis.

In another preferred embodiment, the subject is a patient who has experienced psoriasis recurrence.

In another preferred embodiment, the chemokine receptor CCR6 inhibitor comprises: an extracellular soluble fragment of CCR6, a blocking antibody, a small molecule compound, an antisense nucleic acid, and combinations thereof.

In the second aspect of the present invention, it provides a pharmaceutical product for preventing the recurrence of psoriasis, comprising:
(a) a first pharmaceutical composition comprising (a1) a chemokine receptor CCR6 inhibitor, and (a2) a first pharmaceutically acceptable carrier; and
(b) a second pharmaceutical composition comprising (b1) an additional drug or active ingredient thereof useful for treatment of psoriasis, which is not an CCR6 inhibitor, and (b2) a second pharmaceutically acceptable carrier.

In another preferred embodiment, (b1) the additional drug or active ingredient thereof other than CCR6 inhibitor comprises: glucocorticoid, IL-17A antagonist, TNF-a antagonist, IL-12/IL-23 antagonist, calcineurin inhibitor, vitamin D3 derivative, and combinations thereof.

In another preferred embodiment, in the first pharmaceutical composition, component (a1) accounts for 0.01-99.99 wt%, preferably 0.1-90 wt%, and more preferably 1-80 wt% of the total weight of the first pharmaceutical composition.

In another preferred embodiment, in the first pharmaceutical composition, the concentration of component (a1) is 10 mg/ml-1,000 mg/ml, preferably 20-500 mg/ml, and more preferably 50-200 mg/ml.

In another preferred embodiment, in the second pharmaceutical composition, the component (b1) accounts for 0.01-99.99 wt%, preferably 0.1-90 wt%, and more preferably 1-80 wt% of the total weight of the second pharmaceutical composition.

In another preferred embodiment, in the second pharmaceutical composition, the component (b1) is a glucocorticoid, and accounts for 0.01-50 wt%, preferably 0.01-10 wt%, of more preferably 0.1-5 wt% of the total weight of the second pharmaceutical composition.

In another preferred embodiment, in the second pharmaceutical composition, the component (b1) is an IL-17A antagonist, and has a concentration of 1-1,000 mg/ml, preferably 5-500 mg/ml, and more preferably 10-100 mg/ml in the second pharmaceutical composition.

In another preferred embodiment, the first pharmaceutical composition is liquid, solid, or semi-solid.

In another preferred embodiment, the dosage form of the first pharmaceutical composition is an oral dosage form, an injection, or an topical pharmaceutical dosage form.

In another preferred embodiment, the dosage form of the first pharmaceutical composition comprises tablet, granule, capsule, oral liquid, or injection.

In another preferred embodiment, the first pharmaceutical composition is an oral preparation.

In another preferred embodiment, the carrier is selected from the group consisting of: infusion carrier and/or injection carrier. Preferably, the carrier is one or more carriers selected from the group consisting of: physiological saline, dextrose saline, and combinations thereof.

In another preferred embodiment, the second pharmaceutical composition is liquid, solid, or semi-solid.

In another preferred embodiment, the dosage form of the second pharmaceutical composition is an oral dosage form, an injection, or an topical pharmaceutical dosage form.

In another preferred embodiment, the second pharmaceutical composition is an topical pharmaceutical dosage form.

In another preferred embodiment, the dosage form of the second pharmaceutical composition comprises tablet, granule, capsule, oral liquid, or injection.

In another preferred embodiment, the second pharmaceutical composition is an oral preparation.

In another preferred embodiment, the first pharmaceutical composition and the second pharmaceutical composition in the pharmaceutical product may be made into a same or different dosage form.

In another preferred embodiment, the first pharmaceutical composition and the second pharmaceutical composition in the pharmaceutical product may be administered to the subject simultaneously or sequentially.

It should be understood that within the scope of the present invention, the above-mentioned technical features of the present invention and the technical features specifically described in the following (such as the embodiments) can be combined with each other to form a new or preferred technical solution, which are not redundantly repeated one by one due to space limitation.

### Description of the drawings

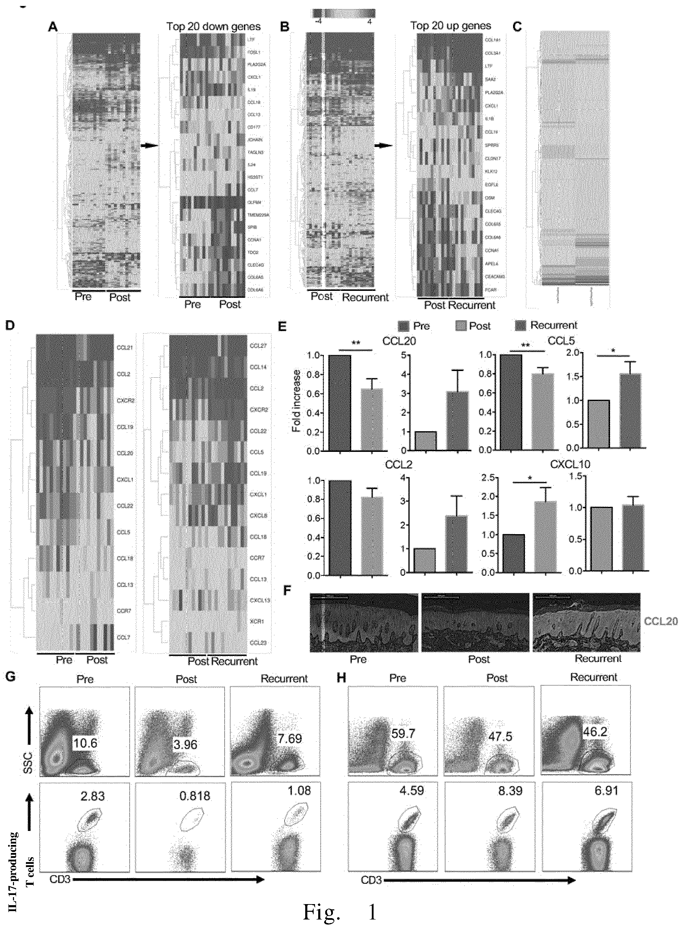

Figure 1 shows results at 3 time points, i.e., before, after treatment with halometasone, and during psoriasis recurrence. Flow cytometry staining showed that the proportion of IL-17-producing cells in peripheral blood increased after treatment and decreased during recurrence; the proportion of IL-17-producing T cells (T17 cells) in skin lesions decreased after treatment and increased during recurrence. Transcriptome sequencing (RNA-seq) showed that the expression of various genes in the skin lesions of psoriasis patients was altered, and especially, the chemokines such as CCL20, CCL5, CCL2, CXCL10, etc., decreased after treatment and increased during recurrence.
Figure 2 shows that in mice treated with halometasone topically for ear skin inflammation, the symptoms of skin psoriasis were significantly relieved, but the proportion of γδT17 cells in the draining and distal lymph nodes was significantly increased.
Figure 3 shows that when psoriasis was re-induced, halometasone-treated mice relapsed rapidly, with a significant decrease in the proportion of γδT17 cells in the draining lymph nodes and distal lymph nodes.
Figure 4 shows the simultaneous treatment of wild-type (WT) and *CCR6*^{*-*/*-*} mice with GC. Compared with WT mice, *CCR6*^{*-*/*-*} mice not only showed reduced symptoms of psoriasis, but also did not show an increase in the proportion of γδT cells in lymph nodes after halometasone treatment.
Figure 5 shows that halometasone could quickly and effectively control psoriasis, but the disease was prone to recurrence. The expression of psoriasis-related genes decreased after treatment, but increased during recurrence.
Figure 6 shows that the proportions of γδT cells and the subpopulations of Vy4 and Vγ4Vδ4 T cells in distal lymph nodes were higher after halometasone treatment, and the proportion of IL-17 secretion after stimulation was also higher.
Figure 7 shows that the proportion of γδT17 cells in lymph nodes of mice in halometasone treatment group was still significantly increased after drug administration was stopped.
Figure 8 shows that the proportion of γδT17 cells in lymph nodes after stimulation of mice in halometasone treatment group was still significantly increased after drug administration was stopped.
Figure 9 shows that in re-induced psoriasis, the proportion of Vγ4 and Vγ4Vδ4 T cells in the skin was higher in the GC group, but there was mild or no change in the lymph nodes.
Figure 10 shows that when psoriasis recurred after GC treatment, CCR6 KO mice still showed milder symptoms of psoriasis compared with wild-type mice, and did not show a decrease of proportion of γδT17 cells in lymph nodes in the GC group.

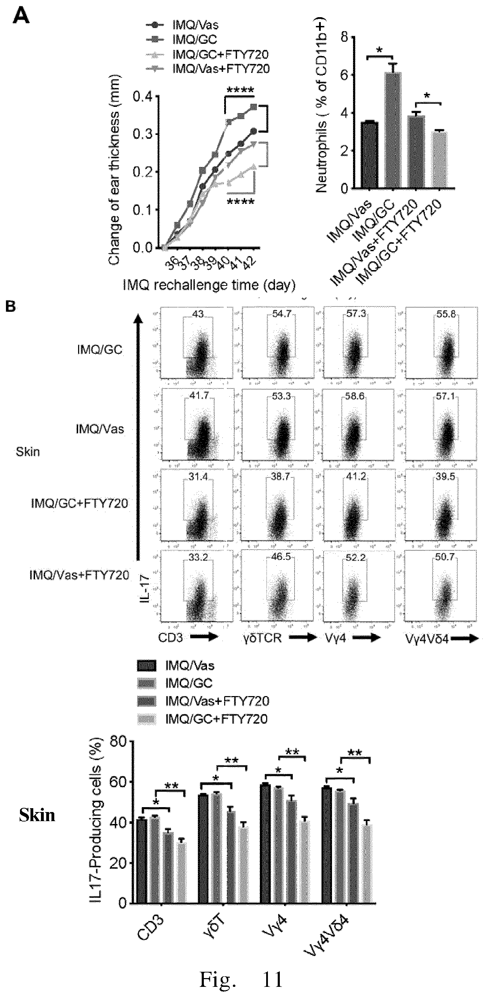

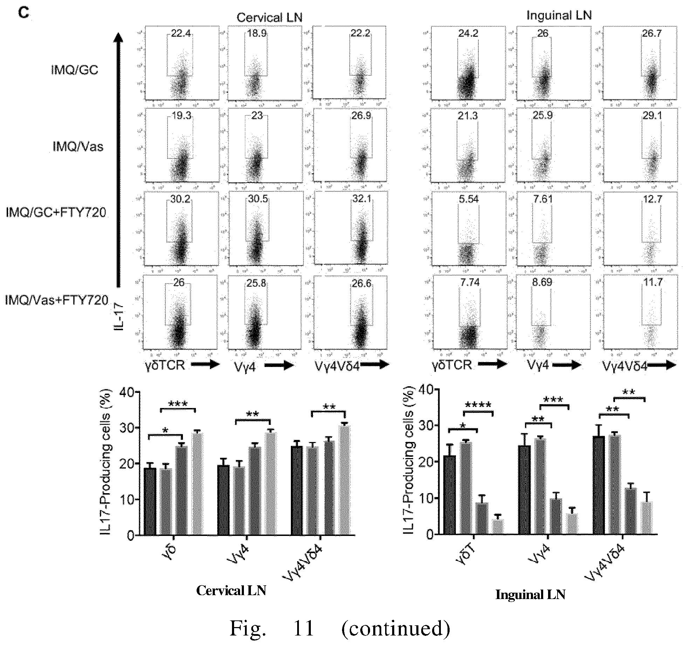

Figure 11 shows that the use of FTY720 prevented γδT17 cells from migrating from the draining lymph nodes, and mice in the GC group did not appear rapid recurrence of psoriasis.

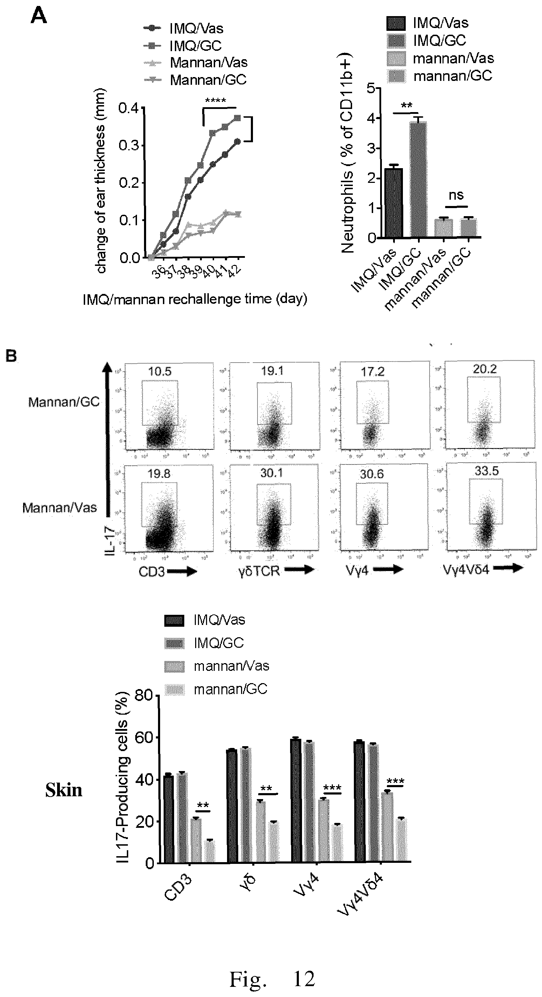

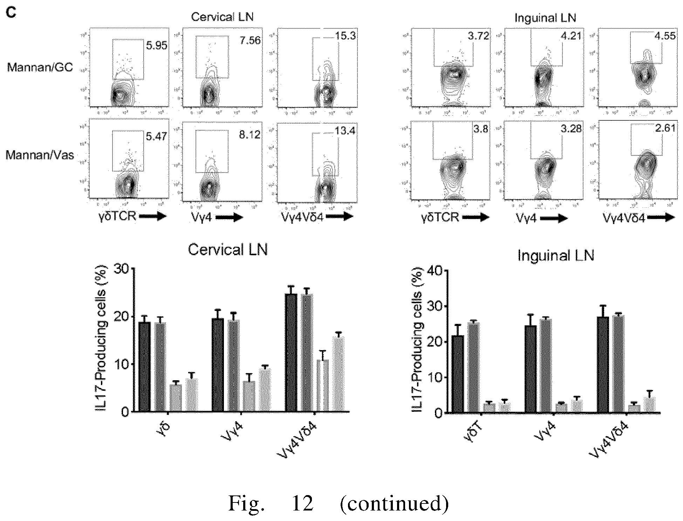

Figure 12 shows that when imiquimod was replaced by mannan for inducing recurrence of psoriasis, the mice in the GC group no longer had rapid disease recurrence.

### Detailed Description

After extensive and intensive research and massive screening, the inventors have developed a method for preventing recurrence of psoriasis for the first time.

Specifically, the inventors utilized an imiquimod (IMQ)-induced mouse psoriasis model to simulate the whole clinical process of topical glucocorticoid treatment in patients with psoriasis: administering of drugs - stop administering - recurrence of disease. It was found that after topical glucocorticoid (GC) treatment, the proportion of γδT cells secreting the pathogenic cytokine IL-17 (γδT17 cells) decreased in the skin, but increased in draining and distant lymph nodes. This change persisted until 2 weeks after GC treatment was discontinued. When IMQ was used again to cause psoriasis recurrence, the previously GC-treated mice exhibited more severe disease recurrence, including more rapid epidermal thickening and increased neutrophil infiltration. Correspondingly, the proportion of γδT17 cells in draining and distant lymph nodes was indistinguishable or even lower compared to the controls.

This indicates that the skin remission after GC treatment is probably due to the migration of γδT17 cells from the skin to the lymph nodes, and when the disease relapses, γδT17 cells can quickly migrate back from the lymph nodes to the skin, leading to the rapid development of psoriasis. That is, GC treatment may lead to psoriasis recurrence by affecting the redistribution of γδT17 cells *in vivo.*

The inventors found that GC treatment in CCR6 knockout mice did not affect the redistribution of γδT17 cells *in vivo* and thus did not lead to psoriasis recurrence. In a further research, it is found that such migratory γδT17 cells have the properties of memory cells. In conclusion, the inventors have found that inhibiting the redistribution of γδT17 cells plays a key role in controlling the recurrence of skin inflammation.

On this basis, the inventors have completed the present invention.

### Pharmaceutical composition and administration method thereof

In the present invention, it provides a pharmaceutical composition comprising a pharmaceutically acceptable carrier and an effective amount of the following active ingredients: a chemokine receptor CCR6 inhibitor and additional drugs or active ingredient thereof for treating psoriasis.

As used herein, the term "effective amount" or "effective dose" refers to an amount that is functional or active in humans and/or animals and is acceptable to humans and/or animals.

As used herein, a "pharmaceutically acceptable" ingredient is a substance that is suitable for use in humans and/or mammals without undue adverse side effects (such as toxicity, irritation, and allergy), i.e., a substance with a reasonable benefit/risk ratio. The term "pharmaceutically acceptable carrier" refers to a carrier for administration of a therapeutic agent, and comprises various excipients and diluents.

The pharmaceutical composition of the present invention comprises a safe and effective amount of the active ingredients of the present invention, and a pharmaceutically acceptable carrier. Such carriers include (but are not limited to) saline, buffers, dextrose, water, glycerol, ethanol, and combinations thereof. Usually, a pharmaceutical preparation should be matched to the administration method, and the pharmaceutical composition of the present invention can be prepared in the form of injection, for example, prepared by conventional methods with normal saline or an aqueous solution containing glucose and other adjuvants. The pharmaceutical composition is preferably manufactured under sterile conditions.

The effective amount of the active ingredients of the present invention may vary with the mode of administration, the severity of the disease to be treated, and the like. Selection of the preferred effective amount can be determined by those skilled in the art based on various factors (e.g., through clinical trials). Such factors include, but are not limited to: pharmacokinetic parameters of the active ingredients such as bioavailability, metabolism, half-life, etc.; severity of the disease to be treated of patients, patient's weights, patient's immune status, route of administration, and the like. For example, several divided doses may be administered daily, or the dose may be proportionally reduced, as dictated by the exigencies of the therapeutic situation.

The pharmaceutically acceptable carrier of the present invention includes (but is not limited to): water, saline, liposomes, lipids, proteins, protein-antibody conjugates, peptides, cellulose, nanogels, and combinations thereof. The choice of carrier should be matched to administration method, as is well known to those skilled in the art.

The first active ingredient (a1) of a chemokine receptor CCR6 inhibitor in the present invention can be used in combination with the second active ingredient (b1) of a additional drug or active ingredient thereof useful for the treatment of psoriasis, which is not a CCR6 inhibitor. The second active ingredient (b1) is an drug already available in the prior art, including but not limited to: glucocorticoids, IL-17A antagonists, TNF-α antagonists, IL-12/IL-23 antagonists, calcineurin inhibitors, vitamin D3 derivatives, and combinations thereof.

### The present invention comprise the following main beneficial effects.

1) Compared with general medicines for treating psoriasis, the present invention has specifically solved the problem of recurrence of psoriasis.
2) It is discovered in the present invention for the first time that the redistribution of pathogenic cells is the mechanism of psoriasis recurrence.
3) The present invention aims at the redistribution characteristics of pathogenic cells in the process of psoriasis recurrence, and has better effect than that of common therapeutic drugs.
4) The present invention can effectively inhibit the redistribution of pathogenic cells, thereby effectively preventing or relieving the recurrence of psoriasis.

The present invention will be further illustrated below with reference to the specific examples. It should be understood that these examples are only to illustrate the invention, not to limit the scope of the invention. The experimental methods with no specific conditions described in the following examples are generally performed under the conventional conditions e.g., the conditions described by Sambrook et al., Molecular Cloning: Laboratory Guide (New York: Cold Spring Harbor Laboratory Press, 1989), or according to the manufacture's instructions. Unless indicated otherwise, all percentage and parts are calculated by weight.

### Example 1: Detection of changes in related gene expression in patients with psoriasis recurrence

In this example, patients with psoriasis vulgaris were recruited and glucocorticoids (GC) were topically (externally) administered for 2 weeks of treatment. Thereafter, the administration were stopped until the disease relapsed, and further research on the mechanism of psoriasis recurrence was carried out.

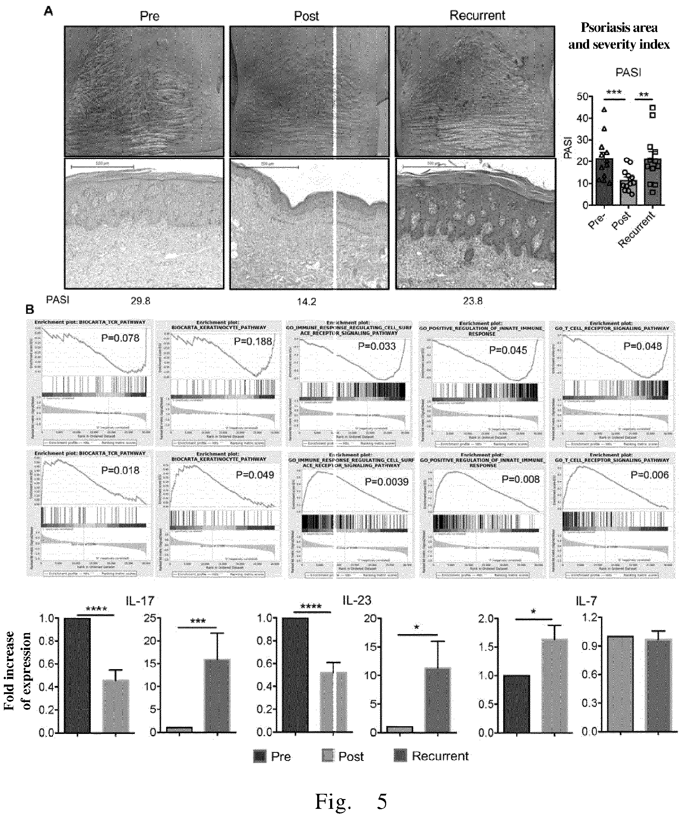

Among the psoriasis patients recruited, 12 patients experienced disease recurrence after an effective GC therapy was stopped (Fig. 5a).

Transcriptome sequencing (RNA-seq) was performed on the skin lesions of these patients before GC treatment, after GC treatment, and after disease recurrence. The results showed that the expression of psoriasis-related genes such as IL-17 and IL-23 was down-regulated after effective GC treatment (Fig. 1a and Fig. 5b). Conversely, the same genes were upregulated when the disease relapsed (Fig. 1b and Fig. 5b).

Comparing gene expression profiles before treatment, after treatment, and during recurrence, it was revealed that up to 400 genes had altered expression (Fig. 1c). Notably, the gene expression changes of multiple chemokines and their receptors were more pronounced (Fig. 1d), including CCL20, CCL5, and CXCL10 (Fig. 1e). Skin tissue immunofluorescence further showed changes in CCL20 expression at different time points (Fig. 1f).

### Example 2: Detection of T cell distribution in patients with psoriasis recurrence

In this example, in psoriasis patients receiving GC treatment, the changes in the proportion of CD3+ T cells and T17 cells in the skin and peripheral blood before treatment, after treatment and during recurrence were detected.

The results showed that after effective GC treatment, the proportion of CD3+ T cells and dermal T17 cells infiltrated in the skin decreased (Fig. 1g), and the proportion of CD3+ T cells and dermal T17 cells in the skin increased when the disease relapsed. In contrast, T17 cells in the blood showed the opposite proportional change, i.e., the proportion increased after treatment and decreased during recurrence (Fig. 1h).

The above study suggests that the redistribution of T cells, especially the redistribution of T17 cells, plays a key role in the remission and recurrence of psoriasis.

### Example 3: Detection of changes in T cell distribution in GC-treated psoriasis animal model

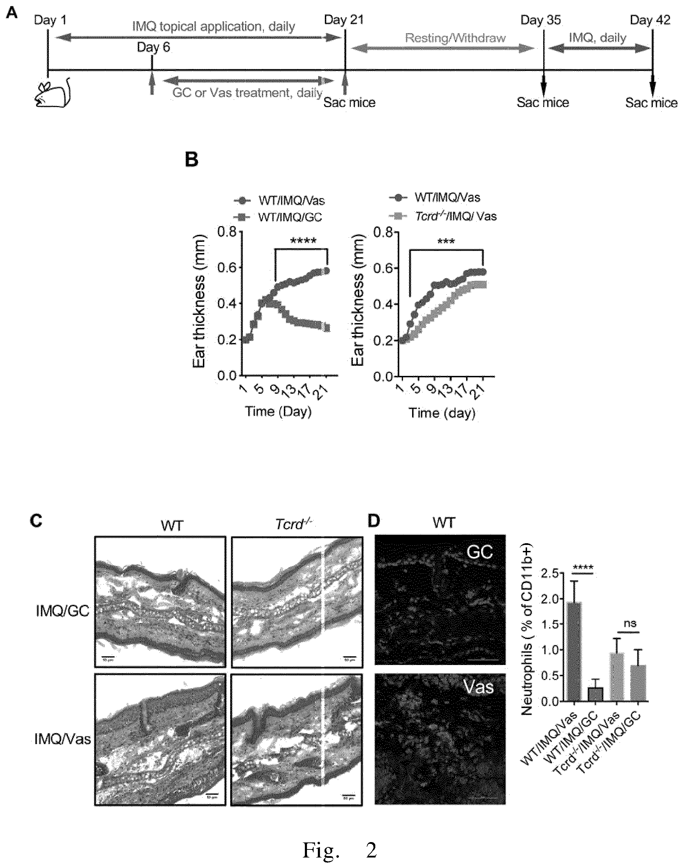

In this example, an animal model to simulate clinical GC treatment for psoriasis was established (Fig. 2a). An imiquimod (IMQ)-induced mouse psoriasis model was utilized to simulate the whole clinical process of topical glucocorticoid treatment in patients with psoriasis: administering of drug - stop administering - recurrence of disease.

All mice were smeared with IMQ for three weeks. Among them, from the 6th day, GC or petrolatum (Vas) was applied to the skin of the mice, and all treatments were stopped on the 21st day. Mice entered a 2-week drug withdrawal period, after which IMQ was applied again for 7 consecutive days to induce psoriasis recurrence.

On day 21, it was seen that the ear thickness of GC-treated mice was significantly thinner than that of the control group (Fig. 2b), which was consistent with clinical remission of psoriasis after GC treatment. Consistent with previous studies, the skin thickness of *TCRd*^{*-*/*-*} mice was significantly thinner than that of normal mice (WT) (Fig. 2b).

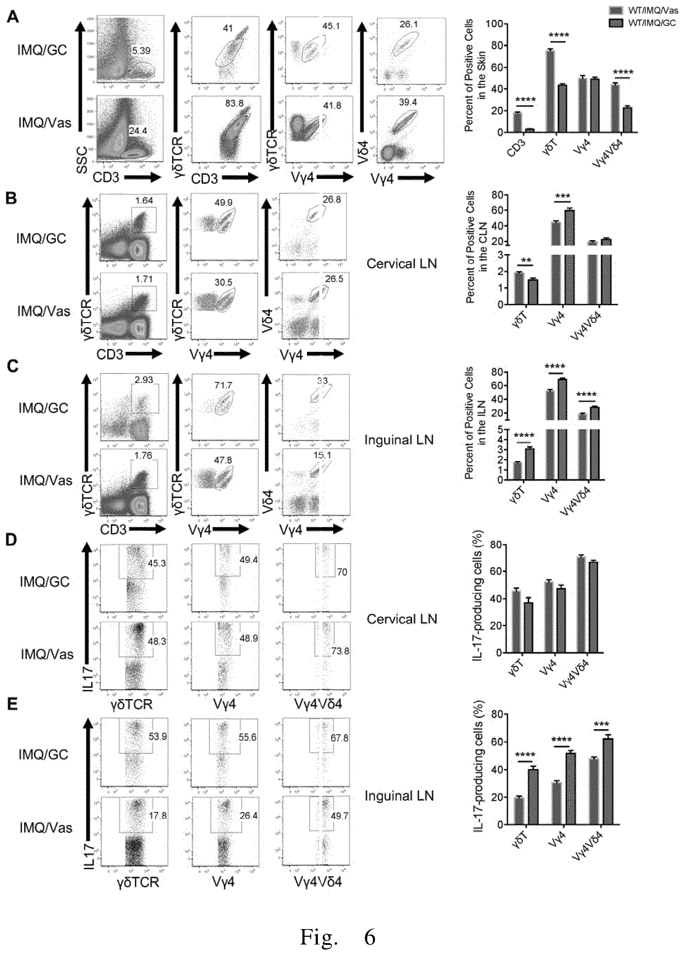

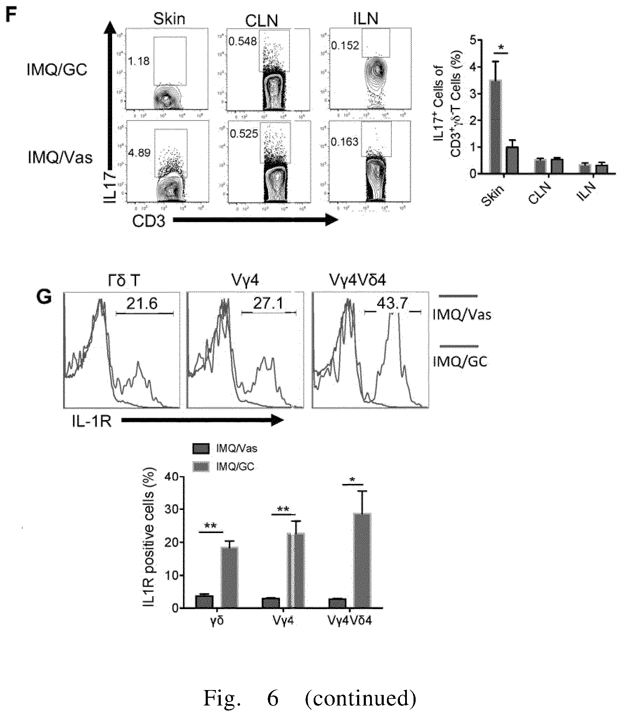

The results of skin H&E staining also confirmed the above results (Fig. 2c). GC treatment also significantly reduced the proportion of CD3+ T cells in the skin (Fig. 6a), which was consistent with the clinical results. Dermal γδT cells, especially Vγ4Vδ4 cells, were significantly reduced in the skin of GC-treated mice (Fig. 6a).

Immunofluorescence and flow staining demonstrated reduced neutrophil infiltration in the GC-treated skin. However, in *TCRd*^{*-*/*-*} mice, there was no statistical difference between the experimental group and the control group.

### Example 4: Detection of changes in IL-17 secretion level in GC-treated psoriasis animal model

γδT cells are the main cells that secrete IL-17 in the skin. In this example, γδT 17 cells in the skin and lymph nodes were detected.

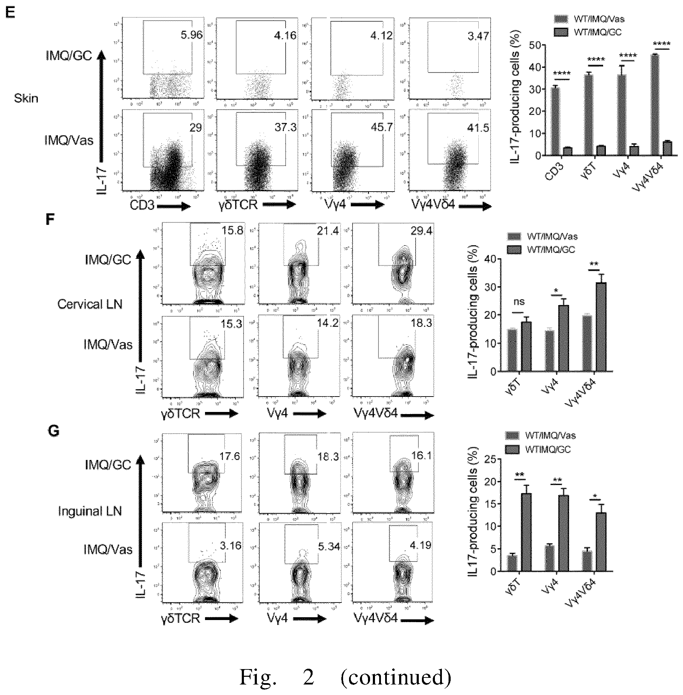

Consistent with disease progression, the proportions of CD3, γδ, Vy4, Vγ4Vδ4 that secreted IL-17 were reduced in the skin of GC-treated mice (Fig. 2e). In skin-draining lymph nodes, although the proportion of γδT cells was decreased in GC treatment group, the proportion of Vγ4 cells was increased (Fig. 6b).

Interestingly, Vy4 cells and Vγ4Vδ4 cells secreted more IL-17 in mice of GC treatment group (Fig. 2f). This change was more significant in distant lymph nodes, wherein the proportions of γδT, Vy4, Vγ4Vδ4 (Fig. 6c), and the proportions of IL-17 secretion thereof (Fig. 2g) were all increased in GC treatment group.

It is noticed that the proportions of IL1R expression in γδT, Vy4, Vγ4Vδ4 cells in the skin were all increased in GC group, and IL1R expression was correlated with the memory ability of Vy4 T17.

These data suggested that γδT17 cells in the skin after GC treatment might migrate to draining and distant lymph nodes, and had a memory cell phenotype.

### Example 5: Detection of changes in yδT17 cell level and IL-17 secretion level in mice during drug withdrawal period

In order to simulate the clinical withdrawal period, all mice in the experimental group and the control group were rested for 2 weeks without treatment.

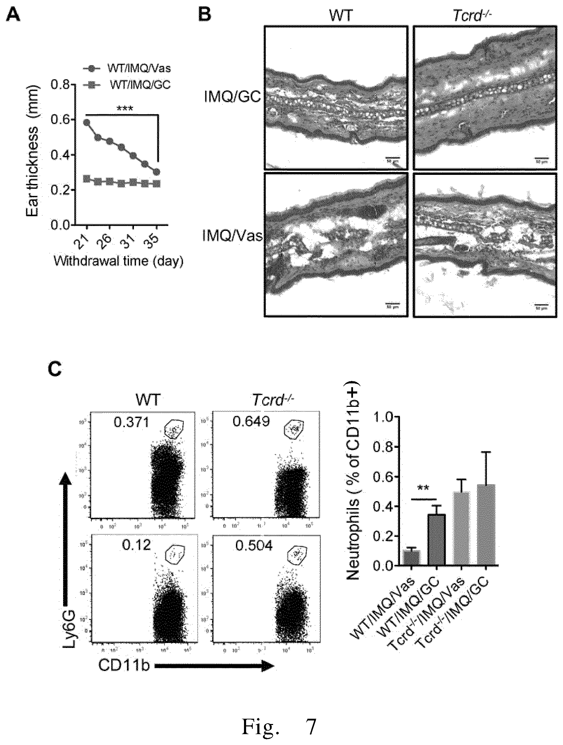

After 2 weeks, the ear thickness of mice in Vas control group gradually recovered (Fig. 7a), and this result was also confirmed by H&E staining (Fig. 7b).

However, more neutrophils were infiltrated in the skin of mice in GC group than in the Vas control group; in *TCRd*^{*-*/*-*} mice, there was no difference between GC group and Vas control group.

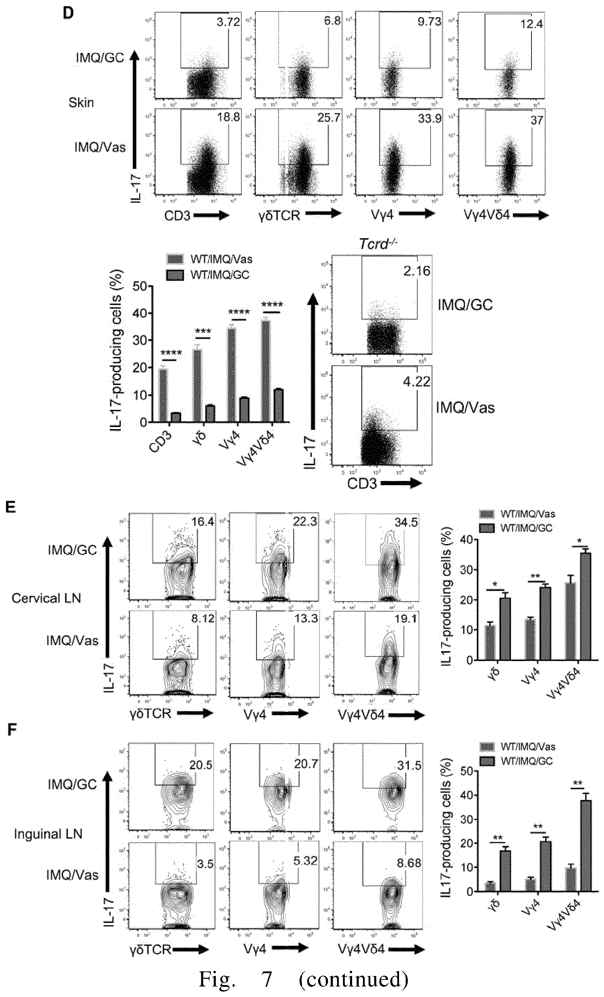

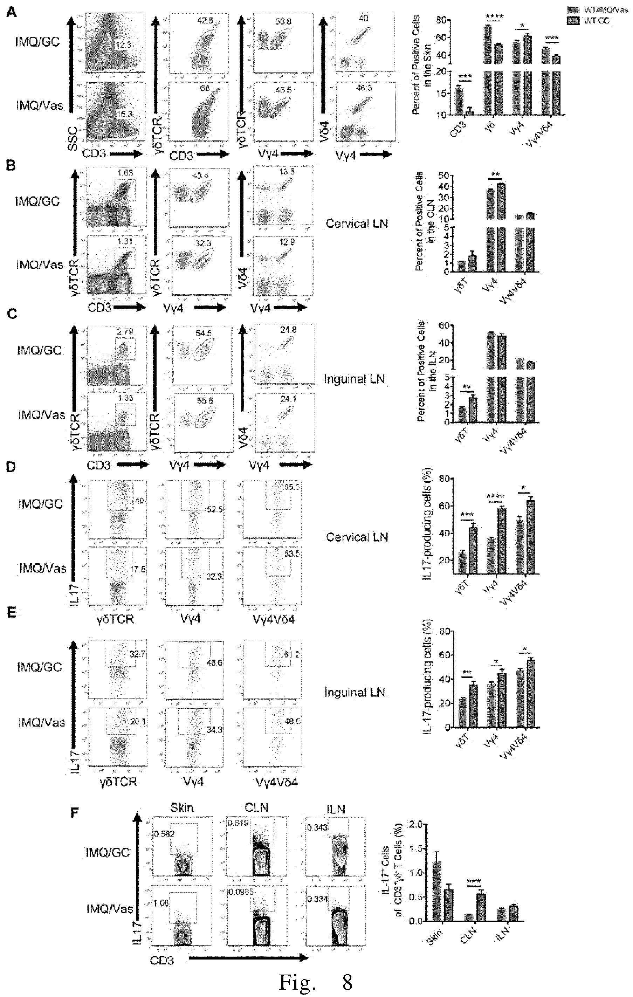

In the skin of mice in GC experimental group, the proportions of CD3, γδT, Vγ4Vδ4 were still lower than those in the control group (Fig. 8a), and the proportion of γδT17 was also lower than that in the control group (Fig. 7d). Although the proportions of γδT, Vy4, Vγ4Vδ4 cells remained unchanged or slightly increased in the draining and distant lymph nodes, the proportion of IL-17-secreting cells was significantly increased (Figs. 7e, 7f).

The above studies showed that 2 weeks after drug withdrawal, although the level of γδT17 cells in the dermis was still low in GC experimental group, the draining and distant lymph nodes still had high levels of γδT17 cells.

### Example 6: Detection of γδT17 cell level and IL-17 secretion level in psoriasis recurrence mouse model

In this example, IMQ was applied to the mice in the experimental group and the control group again to simulate the recurrence of psoriasis after clinical withdrawal.

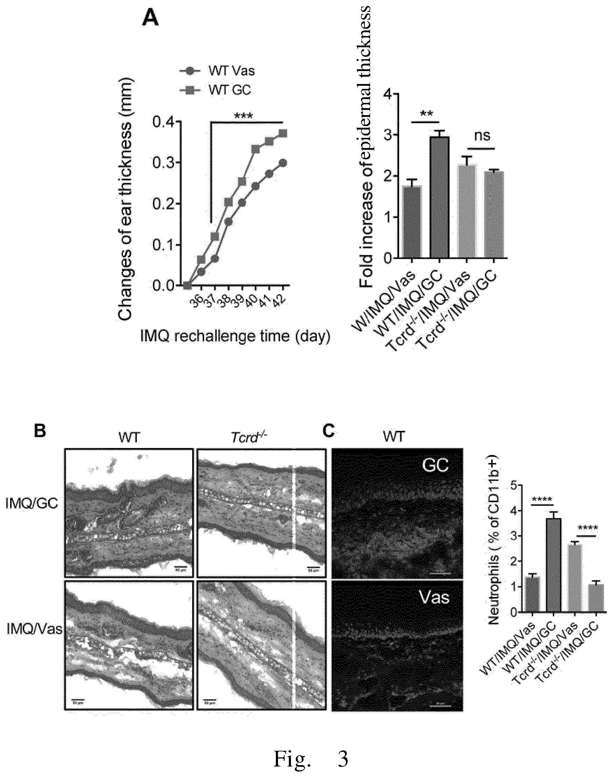

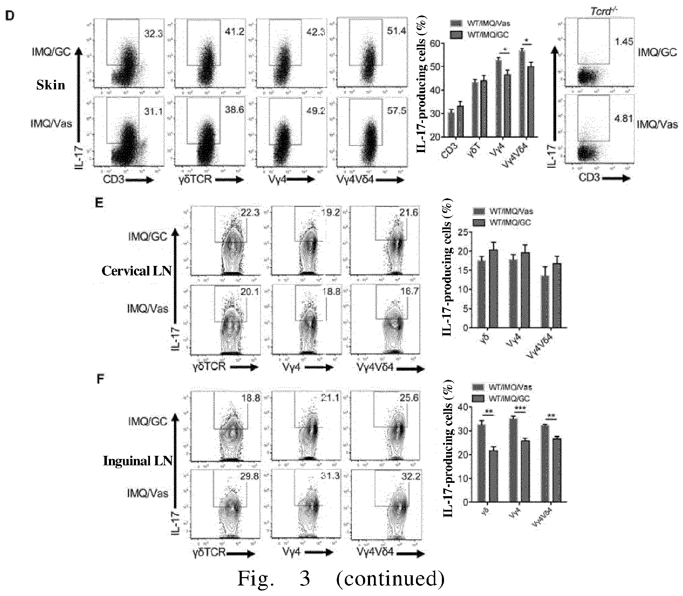

After using IMQ for 3 days, it could be observed that the ear thickening speed of the mice in GC group was faster than that in the control group (Fig. 3a), and the epidermal thickening fold in GC group was also higher than that in Vas group, but there was no difference in *TCRd*^{*-*/*-*} mice between GC group and Vas group (Fig. 3a). H&E staining also confirmed the above results (Fig. 3b).

The infiltrated neutrophils in the skin of mice in GC group exceeded those in Vas control group, while the opposite results were shown in *TCRd*^{*-*/*-*} mice between GC group and Vas group (Fig. 3C).

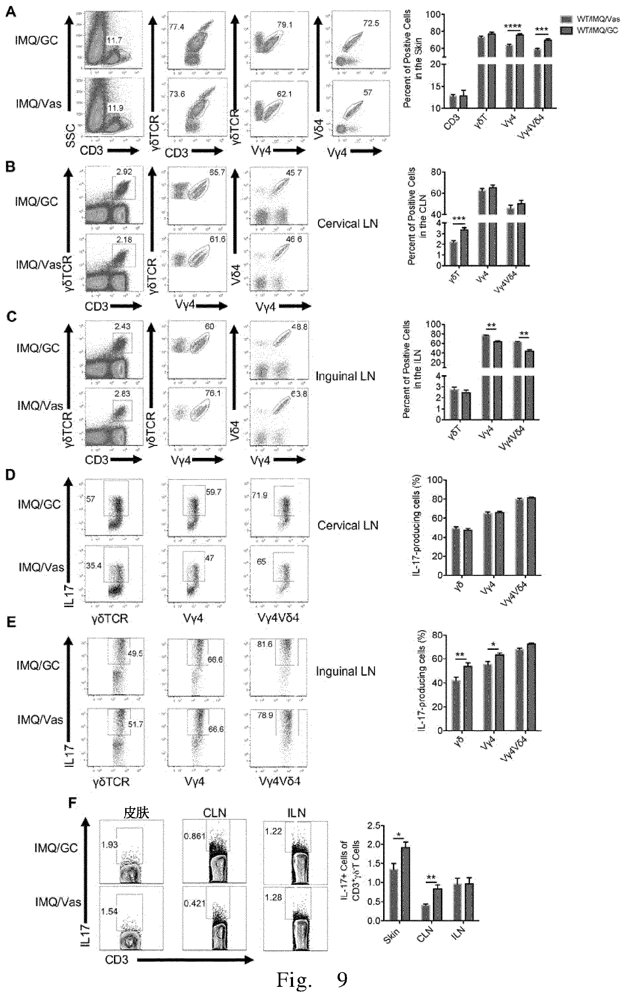

The proportions of CD3 and γδT cells were not significantly different between the two groups, while the proportions of Vy4, Vγ4Vδ4 cells were higher in GC group (Fig. 9a). Although the proportions of Vy4T17 and Vγ4Vδ4T17 cells were slightly decreased in GC group, the levels of IL-17 secreted by CD3 and γδT cells in the skin were not significantly different between the two groups (Fig. 3d).

At draining and distant sites, the proportions of γδT, Vy4, Vγ4Vδ4 cells in lymph nodes varied slightly or did not change between the two groups (Figs. 9b, 9c). There was no difference in γδT17 in skin draining lymph nodes between the two groups, but the proportions of IL-17 secreted by γδT, Vy4, Vγ4Vδ4 cells were significantly lower in the distant lymph nodes of GC group (Fig. 3f). This contrasted sharply with the elevated proportion of IL-17 in draining and distant lymph nodes of GC group before IMQ re-challenge (Fig. 7f).

These studies suggested that elevated proportion of γδT17 in lymph nodes and distant sites would migrate back to the skin when the disease relapsed, leading to rapid skin inflammation.

### Example 7: Study on the relationship between CCR6 gene knockout and yδT17 cell redistribution

Cytokine receptors CCR6 and CCR2 are known to mediate the migration of γδT17 cells in the occurrence of skin inflammation. In this example, the above-mentioned mechanism was studied in wild-type mice (WT), *CCR6*^{*-*/}*⁻,* and *CCR2*^{*-*/*-*} mice.

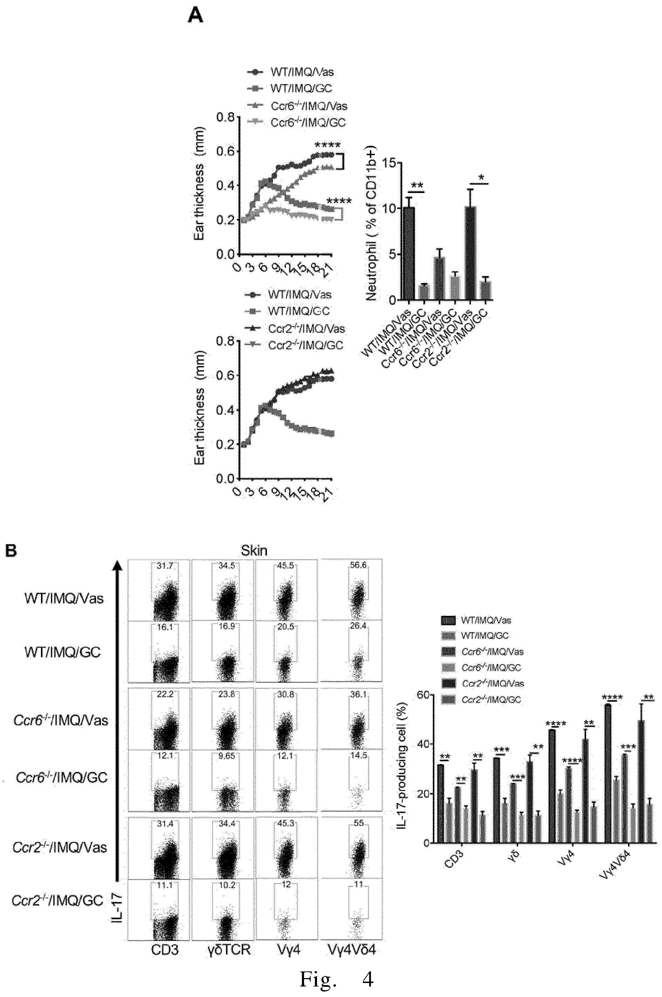

After GC treatment, although the ear thicknesses of the above three strains of mice were reduced, *CCR6*^{*-*/*-*} mice showed differences from WT mice, while *CCR2*^{*-*/*-*} mice showed a similar phenotype as WT mice (Fig. 4a). Both GC-treated WT and *CCR2*^{*-*/*-*} mice showed significantly lower thickness than those in their corresponding controls, but there was no significant difference between *CCR6*^{*-*/*-*} mice and the corresponding control (Fig. 4a).

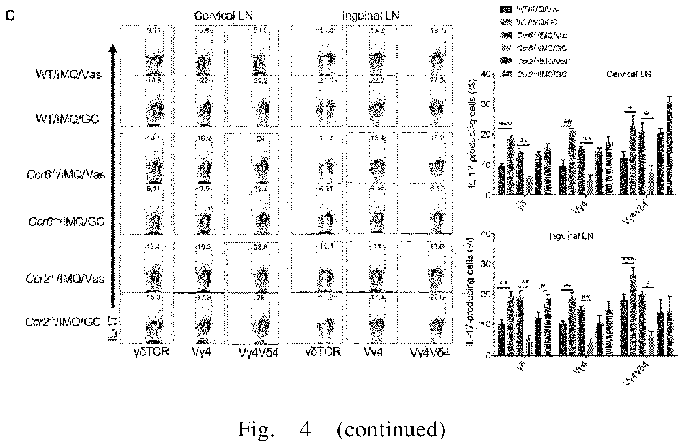

After GC treatment of the three strains of mice, the levels of IL-17 secreted by CD3, γδT, Vy4, Vγ4Vδ4 cells in the skin were all reduced (Fig. 4b), but unlike the WT group and *CCR2*^{*-*/*-*} group, the increased levels of IL-17 secreted by γδT, Vy4, Vγ4Vδ4 cells in draining and distant sites were not observed in *CCR6*^{*-*/*-*} mice (Fig. 4c).

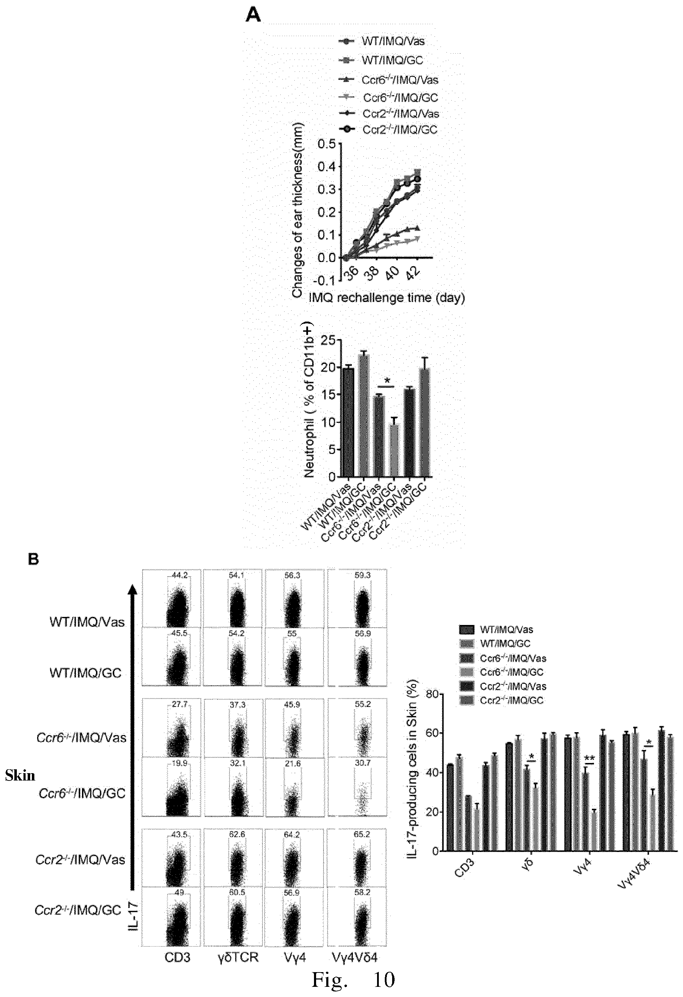

Similarly, WT and CCR2 mice showed faster disease recurrence after re-challenge with IMQ, but the recurrence was significantly suppressed in *CCR6*^{*-*/*-*} mice. *CCR6*^{*-*/*-*} mice exhibited thinner ear thickness (Fig. 10a). Neutrophils also showed similar changes (Fig. 10a).

In the skin, there was no significant difference in the levels of IL-17 secreted by CD3, γδT, Vy4, Vγ4Vδ4 cells between GC treatment group and the corresponding control group of WT and *CCR2⁻¹⁻* mice. However, in GC-treated *CCR6*^{*-*/*-*} mice, the levels of IL-17 secreted by dermal γδT, Vγ4, Vγ4Vδ4 cells were significantly lower than those in the corresponding control group.

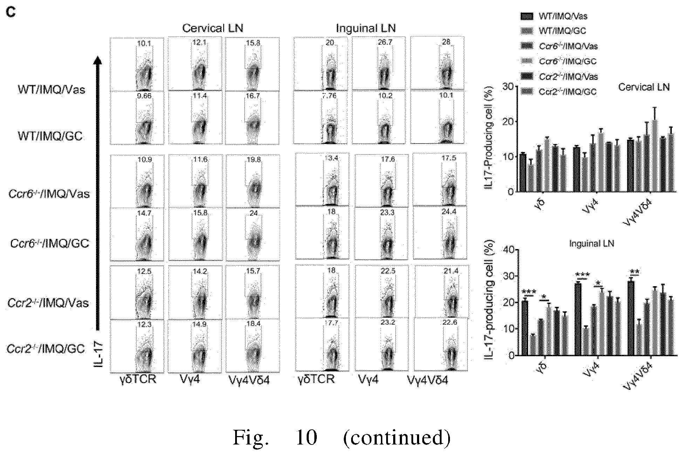

In addition, the proportions of γδT17, Vy4T17, and Vγ4Vδ4T17 cells decreased in draining and distant lymph nodes in WT mice, but not in *CCR6*^{*-*/*-*} mice (Fig. 10c).

The above results indicated that knockout of CCR6 gene would inhibit not only the migration of γδT17 from the skin to the lymph nodes, but also the migration of γδT17 from the lymph nodes to the skin.

## Claims

1. A chemokine receptor CCR6 inhibitor for use in preventing recurrence of psoriasis.

2. The chemokine receptor CCR6 inhibitor for use of claim 1, wherein the chemokine receptor CCR6 inhibitor is administered to a patient with psoriasis.

3. The chemokine receptor CCR6 inhibitor for use of claim 1, wherein the chemokine receptor CCR6 inhibitor is administered to a patient who has experienced psoriasis recurrence.

4. A pharmaceutical product for use in preventing the recurrence of psoriasis, wherein the pharmaceutical product comprises:
(a) a first pharmaceutical composition comprising (a1) a chemokine receptor CCR6 inhibitor, and (a2) a first pharmaceutically acceptable carrier; and
(b) a second pharmaceutical composition comprising (b1) an additional drug or active ingredient thereof useful for treatment of psoriasis, which is not an CCR6 inhibitor, and (b2) a second pharmaceutically acceptable carrier.

5. The pharmaceutical product for use of claim 4, wherein (b1) the additional drug or active ingredient thereof other than CCR6 inhibitor comprises: glucocorticoid, IL-17A antagonist, TNF-a antagonist, IL-12/IL-23 antagonist, calcineurin inhibitor, vitamin D3 derivative, and combinations thereof.

6. The pharmaceutical product for use of claim 4 or 5, wherein in the second pharmaceutical composition, the component (b1) is a glucocorticoid, and accounts for 0.01-50 wt%, preferably 0.01-10 wt%, and more preferably 0.1-5 wt% of the total weight of the second pharmaceutical composition.

7. The pharmaceutical product for use of claim 4 or 5, wherein in the second pharmaceutical composition, the component (b1) is an IL-17A antagonist, and has a concentration of 1-1,000 mg/ml, preferably 5-500 mg/ml, and more preferably 10-100 mg/ml in the second pharmaceutical composition.

## Patentansprüche

1. Chemokinrezeptor-CCR6-Inhibitor zur Verwendung bei einem Vorbeugen eines Wiederauftretens von Psoriasis.

2. Chemokinrezeptor-CCR6-Inhibitor zur Verwendung nach Anspruch 1, wobei der Chemokinrezeptor-CCR6-Inhibitor einem Patienten mit Psoriasis verabreicht wird.

3. Chemokinrezeptor-CCR6-Inhibitor zur Verwendung nach Anspruch 1, wobei der Chemokinrezeptor-CCR6-Inhibitor einem Patienten verabreicht wird, der ein Psoriasis-Wiederauftreten erlebte.

4. Pharmazeutisches Produkt zur Verwendung bei dem Vorbeugen des Wiederauftretens von Psoriasis, wobei das pharmazeutische Produkt umfasst:
(a) eine erste pharmazeutische Zusammensetzung umfassend (a1) einen Chemokinrezeptor-CCR6-Inhibitor und (a2) einen ersten pharmazeutisch verträglichen Träger; und
(b) eine zweite pharmazeutische Zusammensetzung, umfassend (b1) ein zusätzliches Arzneimittel oder einen Wirkstoff davon, das/der für eine Behandlung von Psoriasis nützlich ist, das/der kein CCR6-Inhibitor ist, und (b2) einen zweiten pharmazeutisch verträglichen Träger.

5. Pharmazeutisches Produkt zur Verwendung nach Anspruch 4, wobei (b1) das zusätzliche Arzneimittel oder der Wirkstoff davon, außer dem CCR6-Inhibitor, umfasst: Glucocorticoid, IL-17A-Antagonist, TNF-α-Antagonist, IL-12/IL-23-Antagonist, Calcineurin-Inhibitor, Vitamin-D3-Derivat und Kombinationen davon.

6. Pharmazeutisches Produkt zur Verwendung nach Anspruch 4 oder 5, wobei in der zweiten pharmazeutischen Zusammensetzung die Komponente (b1) ein Glucocorticoid ist und 0,01-50 Gew.-%, vorzugsweise 0,01-10 Gew.-% und mehr bevorzugt 0,1-5 Gew.-% des Gesamtgewichts der zweiten pharmazeutischen Zusammensetzung ausmacht.

7. Pharmazeutisches Produkt zur Verwendung nach Anspruch 4 oder 5, wobei in der zweiten pharmazeutischen Zusammensetzung die Komponente (b1) ein IL-17A-Antagonist ist und eine Konzentration von 1-1.000 mg/ml, vorzugsweise 5-500 mg/ml und mehr bevorzugt 10-100 mg/ml in der zweiten pharmazeutischen Zusammensetzung aufweist.

## Revendications

1. Inhibiteur du récepteur de chimiokine CCR6 pour une utilisation dans la prévention de la récidive du psoriasis.

2. Inhibiteur du récepteur de chimiokine CCR6 pour une utilisation selon la revendication 1, dans lequel l'inhibiteur du récepteur de chimiokine CCR6 est administré à un patient atteint de psoriasis.

3. Inhibiteur du récepteur de chimiokine CCR6 pour une utilisation selon la revendication 1, dans lequel l'inhibiteur du récepteur de chimiokine CCR6 est administré à un patient qui a présenté une récidive de psoriasis.

4. Produit pharmaceutique pour une utilisation dans la prévention de la récidive du psoriasis, dans lequel le produit pharmaceutique comprend :
(a) une première composition pharmaceutique comprenant (a1) un inhibiteur du récepteur de chimiokine CCR6, et (a2) un premier support pharmaceutiquement acceptable ; et
(b) une seconde composition pharmaceutique comprenant (b1) un médicament supplémentaire ou un principe actif de celui-ci utile pour le traitement du psoriasis, qui n'est pas un inhibiteur de CCR6, et (b2) un second support pharmaceutiquement acceptable.

5. Produit pharmaceutique pour une utilisation selon la revendication 4, dans lequel (b1) le médicament supplémentaire ou le principe actif de celui-ci autre qu'un inhibiteur de CCR6 comprend : glucocorticoïde, antagoniste de l'IL-17A, antagoniste du TNF-a, antagoniste de I'IL-12/IL-23, inhibiteur de la calcineurine, dérivé de la vitamine D3, et combinaisons de ceux-ci.

6. Produit pharmaceutique pour une utilisation selon la revendication 4 ou 5, dans lequel, dans la seconde composition pharmaceutique, le composant (b1) est un glucocorticoïde, et représente 0,01 à 50 % en poids, de préférence 0,01 à 10 % en poids, et plus préférablement 0,1 à 5 % en poids du poids total de la seconde composition pharmaceutique.

7. Produit pharmaceutique pour une utilisation selon la revendication 4 ou 5, dans lequel dans la seconde composition pharmaceutique, le composant (b1) est un antagoniste de l'IL-17A, et a une concentration de 1 à 1000 mg/ml, de préférence 5 à 500 mg/ml, et plus préférablement 10 à 100 mg/ml dans la seconde composition pharmaceutique.
